# EUROPÄISCHE PATENTSCHRIFT

(11) **EP 1 681 014 B1**
(45) Veröffentlichungstag und Bekanntmachung des Hinweises auf die Patenterteilung: **05.10.2011**
(21) Anmeldenummer: 06000684.8
(22) Anmeldetag: 13.01.2006
(51) Int. Cl.: A61B 1/31, A61B 1/273, A61B 19/00, A61M 25/01

(54) **Antrieb für ein Endoskop**
drive unit for an endoscope
entraînement pour un endoscope

(30) Priorität: 18.01.2005 DE 102005002461
(43) Veröffentlichungstag der Anmeldung: 19.07.2006
(73) Patentinhaber: Neumann, Martin, Dr., 91080 Uttenreuth (DE)
(72) Erfinder: Neumann, Dr. Martin, 91080 Uttenreuth (DE); Bayer, Karl-Heinz, 91077 Kleinsendelbach (DE)
(74) Vertreter: Gosdin, Michael

(56) Entgegenhaltungen:
- DE-A1- 19 920 717
- US-A- 5 540 649
- US-A1- 2004 097 789
- US-B1- 6 171 234

## Beschreibung

Die Erfindung betrifft einen Antrieb für ein zumindest abschnittsweise rohrförmig ausgebildetes, flexibles Endoskop zu dessen Bewegung in axialer Richtung, wobei der Antrieb des Endoskops mindestens zwei Trägerelemente aufweist, die relativ zueinander senkrecht zur axialen Richtung des Endoskops in einer Verstellrichtung bewegbar und in einer gewünschten Position feststellbar sind, wobei auf jedem der mindestens zwei Trägerelemente je mindestens eine Antriebswalze angeordnet ist, deren Achse im wesentlichen senkrecht zur axialen Richtung des Endoskops und im wesentlichen senkrecht zur Verstellrichtung angeordnet ist, wobei die mindestens eine Antriebswalze zum reibschlüssigen Antrieb des zwischen den Antriebswalzen angeordneten Endoskops ausgebildet sind, und wobei zumindest eine der Antriebswalzen, vorzugsweise alle Antriebswalzen, von einem steuer- oder regelbaren Elektroantrieb angetrieben wird, wobei der Elektroantrieb mit einem Pedal in Verbindung steht, über dessen Betätigungsweg und/oder Betätigungsrichtung die Drehgeschwindigkeit und/oder Drehrichtung der Antriebswalzen beeinflusst wird.

Für diagnostische und therapeutische Untersuchungseingriffe werden flexible Endoskope eingesetzt, die im Stand der Technik hinlänglich bekannt sind. Sie werden rektal oder oral eingeführt und an die zu untersuchende Stelle bzw. entlang des zu untersuchenden Bereichs geschoben, z. B. in den Bereich des Dickdarms (Koloskopie), des Zwölffingerdarms, des Magens (Gastroskopie) oder der Speiseröhre.

Die Richtungssteuerung der in zwei Ebenen bewegbaren Endoskopspitze erfolgt dabei mechanisch mittels zwei Steuerräder am Endoskophandgriff, um möglichst lumenzentriert und die Darmwand schonend vorzuspiegeln oder um eine Zielregion optimal einzustellen. Der Vorschub bzw. die Rückwärtsbewegung des Endoskops wird dabei manuell durchgeführt, wobei eine Hand des Untersuchers die Steuerräder am Endoskophandgriff loslassen muss, um den Endoskopschaft zu greifen und vorzuschieben. Damit sich der Untersucher auf die wichtigen Funktionen im Handgriffbereich des Endoskops - zwei Steuerräder und die Instrumentenbedienung an der Öffnung des Arbeitskanals - konzentrieren kann, wird diese Funktion häufig auch vom Endoskopie-Assistenzpersonal übernommen. Ein wichtiger Punkt beim Vorspiegeln ist es, die Darmwand möglichst zu schonen, um Schmerzen für den Patienten zu vermeiden, und um Verletzungen an der Darmwand - im schlimmsten Fall eine Darmperforation (Durchbruch) - zu vermeiden.

Ein Endoskopantrieb der eingangs genannten Art ist aus der US 5,779,623 bekannt. Mit dem dort beschriebenen Antrieb kann ein Endoskop in axiale Richtung vorgeschoben werden, wobei eine Steuerung des Antriebs durch Pedale erfolgen kann, die vom Operateur entsprechend betätigt werden.

Ein ähnliche Antrieb für ein Endoskop ist aus der US 2004/0097789 A1 bekannt. In der US 6,726,675 ist ein Antrieb beschrieben, mit dem ein Katheter in seine Achsrichtung in den Körper eines Patienten vorgeschoben werden kann. Um Schäden bei der Endoskopie an der Darmwand zu vermeiden, ist es aus der DE 42 42 291 A1 und aus der DE 199 20 717 A1 bekannt, einen Stülpschlauch einzusetzen, dessen äußerer Bereich relativ zur Darmwand ruht und der innere Bereich die Bewegung des Endoskops aufnimmt. Für eine optimale Anpassung an das unterschiedlich weite Darmlumen kann mit diesem System auch mittels Flüssigkeit das Volumen zwischen der äußeren und inneren Stülpschlauchwand verändert werden. Für dieses Stülpschlauchsystem wird auch eine Antriebseinheit allgemein in Form von elektronisch antreibbaren Antriebsrädern offenbart, die in einem Gehäuse um das Stülpschlauchsystem angeordnet sein können und eine Antriebskraft auf den Stülpschlauch und das Endoskop übertragen.

Weiterhin ist ein Führungssystem für Endoskope aus der DE 101 41 226 A1 bekannt, das aus einem Gehäuse besteht, welches konzentrisch um den Endoskopschaft angeordnet ist, wobei drehbar gelagerte Antriebshülsen sowohl eine transversale Bewegung als auch eine rotatorische Bewegung des Endoskopschaftes ermöglichen.

Die DE 101 41 225 A1 zeigt ein einen mechanischen Halte- und Führungsarm aufweisendes Endoskopführungssystem, das aus mehreren Gelenkverbindungen, einer Parallelogrammführung und Antriebseinheiten besteht.

Gerade bei schwierigen Untersuchungsverhältnissen kommt es beim Vorspiegeln sehr darauf an, die Vorschubbewegung des Endoskops feinfühlig zu bewerkstelligen und dabei eine Kontrolle über die für den Vorschub erforderlichen Kräfte zu haben, um unnötige Schmerzen, Schäden an der Darmwand oder gar Darm-Perforationen zu vermeiden.

Diese Risikofaktoren und die individuelle Einschätzung, ab wann die Vorschubkraft gefährlich werden könnte, stehen im direkten Zusammenhang mit der praktische Erfahrung und Lernkurve des jeweiligen Untersuchers.

Die im Stand der Technik beschriebenen Vorrichtungen bieten diesbezüglich noch keine optimale Lösung. Vielmehr bleibt es in der Regel erforderlich, das Endoskop zumindest in kritischen Bereichen manuell vorzuschieben, um Verletzungen des Patienten ausschließen zu können.

Wenn die Funktion des Vorschiebens und Zurückziehens des Endoskops vom Untersucher selbst übernommen wird, muss er in diesem Moment eine Hand von den Steuerrädern am Endoskop-Handgriff nehmen und den Prozess der Richtungssteuerung der Endoskopspitze unterbrechen. Vor allem auch bei interventionellen endoskopischen Eingriffen, das heißt, wenn über den Arbeitskanal im Handgriffbereich des Endoskops Instrumente, beispielsweise Biopsiezangen oder Injektionsnadeln, mit einer Hand bedient werden müssen, stört es sehr, wenn zusätzlich für die Feinsteuerung und das genaue Anfahren des Endoskops an die pathologische Darmwandregion eine Hand das Schieben des Endoskops übernehmen muss. Wird das Vor- und Rückbewegen des Endoskops vom Assistenzpersonal übernommen, ist es eine wichtige Voraussetzung, dass das die Untersuchung durchführende Team (Arzt - Assistenzpersonal) gut eingespielt ist und vor allem die Kommunikation zwischen den Personen gut funktioniert.

Nachteilig ist es dabei allerdings auch, dass die Assistenzperson während des Einführens des Endoskops gebunden ist und für andere Aufgaben, wie Betreuung / Überwachung des Patienten, Vorbereiten von Instrumenten, etc., nicht zur Verfügung steht.

Der Erfindung liegt im Lichte der vorstehenden Probleme und Nachteile daher die A u f g a b e zugrunde, einen Endoskopantrieb der gattungsgemäßen Art so weiterzuentwickeln, dass die genannten Nachteile vermieden werden können. Es soll also die Möglichkeit geschaffen werden, die Vorschub- und Rückziehbewegung des Endoskops feinfühlig und überwacht zu bewerkstelligen, so dass Verletzungen des Patienten ausgeschlossen werden können. Dennoch soll dem untersuchenden Arzt die Möglichkeit gegeben werden, zur optimalen Richtungssteuerung der Endoskopspitze seine Hände an den Steuerrädern des Endoskophandgriffs belassen zu können.

Die L ö s u n g dieser Aufgabe durch die Erfindung ist dadurch gekennzeichnet, dass die mindestens zwei Trägerelemente relativ zueinander mittels eines Schrauben-Gewinde-Elements bewegbar und/oder einstellbar sind, wobei das Schrauben-Gewinde-Element eine Entraststellung oder Entrastmittel aufweist, in der die oder mit der die mindestens zwei Trägerelemente ohne Betätigung des Schrauben-Gewinde-Elements voneinander entfernbar sind.

Mit dieser Ausgestaltung wird es möglich, bei Bedarf sehr schnell auf üblichen Handvorschub des Endoskops umschwenken zu können, falls dies aufgrund spezieller Umstände sinnvoll oder notwendig werden sollte. Dann wird das Endoskop aus der Klemmung durch die Antriebswalzen entkoppelt und händisch manipuliert.

Mit dieser Ausgestaltung wird es also in besonders einfacher Weise möglich, dass insbesondere das Einführen des Endoskops in Körperhöhlen sehr feinfühlig erfolgen kann, wobei dennoch sichergestellt ist, dass der Arzt beide Hände am Steuerungshandgriff des Endoskops belassen kann.

Eine erste Weiterbildung sieht vor, dass der Antrieb zur Bewegung des Endoskops in beide Achsrichtungen ausgebildet ist.

Das Pedal und/oder der steuer- oder regelbare Elektroantrieb kann zur stufenlosen Bewegung des Endoskops ausgebildet sein.

Um eine Verletzung des Patienten durch das Endoskop infolge zu hoher Vorschubkraft auszuschließen, können Kraftmessmittel vorgesehen werden, mit denen die von der mindestens einen Antriebswalze auf das Endoskop aufgebrachte axiale Kraft messbar ist. Die Kraftmessmittel können mindestens einen Dehnmessstreifen aufweisen. Ferner können Anzeigemittel zum Anzeigen der von der mindestens einen Antriebswalze auf das Endoskop aufgebrachten axialen Kraft vorgesehen werden. Die Anzeigemittel können dabei optische Elemente aufweisen, insbesondere Leuchtdioden, die bereichsweise unterschiedliche Farben haben. Damit kann in einfacher Weise und übersichtlich überwacht werden, wie hoch die Vorschubkraft ist. Die Anzeigemittel können auch bzw. alternativ akustische Elemente aufweisen, um akustisch auf ein unzulässig hohes Ansteigen der Vorschubkraft hinweisen zu können. Als Alternative kommt auch in Frage, dass die Anzeigemittel Vibrationen aussendende Elemente aufweisen, mittels der auf steigende Vorschubkräfte aufmerksam gemacht wird. Die Kraftmessmittel können mit einem Schaltelement in Verbindung stehen, das zur Abschaltung des Elektroantriebs bei Überschreitung eines vorgegebenen Maximalwertes der von der mindestens einen Antriebswalze auf das Endoskop aufgebrachten axialen Kraft ausgebildet ist.

Das Schrauben-Gewinde-Element kann dabei zur spiegelbildlichen Betätigung der mindestens zwei Trägerelemente zu einer Symmetrieebene ausgebildet sein.

Die Antriebswalzen können eine Ummantelung mit einem Material mit hohem Reibungskoeffizienten aufweisen, wobei insbesondere an Gummi gedacht ist. Die Walzen haben vorzugsweise einen konischen Außenumfang. Je zwei zusammenwirkende Antriebswalzen können dabei so angeordnet sein, dass ihre konischen Außenumfänge gegensinnig orientiert sind.

Das Pedal kann mit zwei Fußtritten ausgestattet sein, wobei einer für den Vortrieb des Endoskops in die eine axiale Richtung und einer für den Rückzug des Endoskops in die andere axiale Richtung vorgesehen ist.

Die Trägerelemente samt Antriebswalzen sind vorzugsweise frei zugänglich. Zumindest die Antriebswalzen können mit der restlichen Antriebsvorrichtung koppelbar und von dieser entkoppelbar ausgebildet sein. Die Antriebswalzen können insbesondere auf einem Modul angeordnet sein. Mit dieser Ausgestaltung kann in einfacher Weise erreicht werden, dass die Walzen - beispielsweise über Schnellkupplungsmittel - von dem Antrieb entnommen und gereinigt werden können. Unter dem Modul kann eine Auffangschale angeordnet sein, die zu den Trägerelementen abgedichtet ist. Auch hierdurch wird eine einfache Reinigung und hygienische Handhabung des gesamten Antriebs vereinfacht. Sinnvoll sind auch Waschmittel zum Reinigen des Endoskops.

Der Antrieb kann schließlich Wegmessmittel zum Messen des Vorschub- und Rückzugswegs des Endoskops aufweisen.

Der Erfindungsvorschlag stellt also darauf ab, dass für handelsübliche flexible Endoskope unterschiedlicher Durchmessergrößen die Vor- und Rückbewegung bei diagnostischen und/oder therapeutischen Untersuchungseingriffen im Darm / Speiseröhre / Magen / Zwölffingerdarm durch einen elektronisch geregelten Antrieb mit Fußsteuerung bewerkstelligt wird, mit der variable Geschwindigkeiten sowohl in Einführrichtung als auch in Ausführrichtung stufenlos regulierbar sind, wobei die Vorschubkraft messbar und darstellbar ist, und es bei der Überschreitung einer vorwählbaren Grenze für die Kraft zu einer automatischen Sicherheitsabschaltung der Vorwärtsbewegung des Endoskops kommt.

Vorgesehen ist es hierzu, das manuelle Vorschieben und Zurückführen der handelsüblichen Endoskope bei der Untersuchung durch einen fußgesteuerten elektrischen Antrieb zu ersetzen, der vom Untersucher selbst bedient werden kann, damit er sich für die Steuerung und Bedienung der Instrumente im Handgriffbereich konzentrieren kann. Ferner ergibt es sich durch den Erfindungsvorschlag, dass sich der Assistent auf andere wichtige Aufgaben konzentrieren kann.

Durch die Abschaltung des Endoskopantriebs bei Überschreitung einer vorgegebenen Vorschubkraft bzw. bei der Ausgabe entsprechender Signale bei ansteigender Vorschubkraft können individuelle Erfahrungswerte bezüglich der vertretbaren Vorschubkraft bei der Endoskopie auch für jüngere Ärzte objektivierbar und als Richtwert deutlich gemacht werden. Mit den ermittelten Werten können zudem optimale Einstellungen bezüglich der Schmerzbelastung für den Patienten ermittelt und berücksichtigt werden.

Es können handelsübliche, flexible Endoskope mit unterschiedlichen Durchmessern eingesetzt werden, wobei keine spezielle Vorbereitungen oder Ausstattungen der Seiten des Endoskops erforderlich sind. Die Antriebswalzen kontaktieren das Endoskop kraftschlüssig und ohne Beschädigung dessen Ummantelung.

In der Zeichnung ist ein Ausführungsbeispiel der Erfindung dargestellt. Es zeigen:
- Fig. 1: die Seitenansicht des Antriebs für ein Endoskop zu dessen Bewegung in axialer Richtung,
- Fig. 2: die zu Fig. 1 korrespondierende Draufsicht,
- Fig. 3: die zu Fig. 1 korrespondierende Vorderansicht im Schnitt (Schnitte C-D und E-F gemäß Fig. 1 und A-B gemäß Fig. 2),
- Fig. 4: die zu Fig. 1 korrespondierende Vorderansicht im Schnitt (Schnitte G-H und I-K gemäß Fig. 2) und
- Fig. 5: das Pedal des Antriebs in perspektivischer Darstellung.

In den Figuren ist ein Antrieb 1 für ein Endoskop 2 dargestellt, das entlang seiner axialen Richtung a in beide Achsrichtungen bewegt werden kann. Der Antrieb hat zwei Trägerelemente 3 und 4, die spiegelbildlich zu einer Symmetrieebene 15 des Antriebs 1 relativ zum Grundgerüst 23 des Antriebs 1 beweglich sind. Die spiegelbildliche Bewegung beider Trägerelemente 3, 4 wird durch ein Schrauben-Gewinde-System 14 bewerkstelligt, das eine Gewindespindel 24 mit Rückstellfeder (die die beiden Trägerelemente 3, 4 auseinander drückt) und mit zwei Gewindeabschnitten aufweist, wobei beide Gewindeabschnitte gegensinnig gerichtet sind, d. h. der eine Gewindeabschnitt ist als Recht- und der andere als Linksgewinde ausgeführt. Die Gewindeabschnitte wirken mit Gewindemuttern zusammen, die mit den Trägerelementen 3, 4 verbunden sind.

Dies hat zur Folge, dass durch Drehung der Gewindespindel 24 mittels eines Handrads 25 die beiden Trägerelemente 3, 4 aufeinander zu bzw. voneinander weg bewegt werden. Diese Verstellbewegung erfolgt in eine Richtung s, die senkrecht zur axialen Richtung a liegt, die durch das Endoskop 2 definiert ist, wenn dieses im Antrieb 1 ist.

Auf den Trägerelementen 3, 4 sind insgesamt vier Antriebswalzen 5, 6, 7 und 8 angeordnet, wobei die Achse 9 der Walzen 5, 6, 7, 8 senkrecht auf der axialen Richtung a und senkrecht auf der Verstellrichtung s steht.

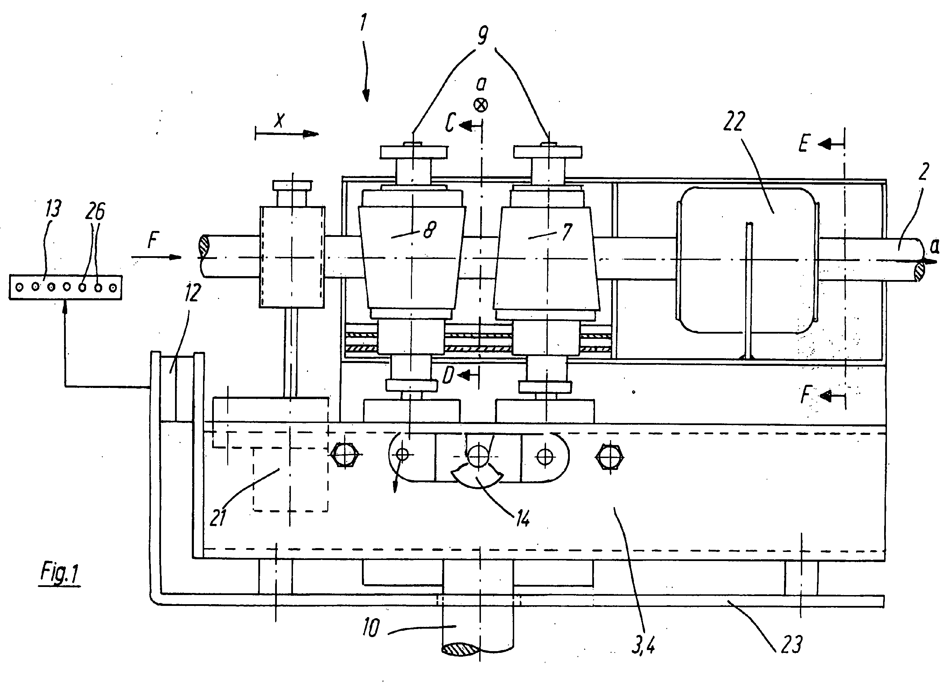

Wie aus der Zusammenschau der Figuren 1, 2 und 3 ersichtlich ist, sind zwei Walzen 5 und 6 auf dem Trägerelement 4 und zwei Walzen 7 und 8 auf dem Trägerelement 3 angeordnet. Alle Walzen weisen einen Außenumfang 16 auf, der einen konischen Verlauf hat. Das Endoskop 2 wird von je zwei Walzen auf jedem der Trägerelemente 3, 4 mittels Reibschluss geklemmt, so dass bei der Drehung der Walzen 5, 6, 7, 8 das Endoskop 2 in axiale Richtung a verschoben wird.

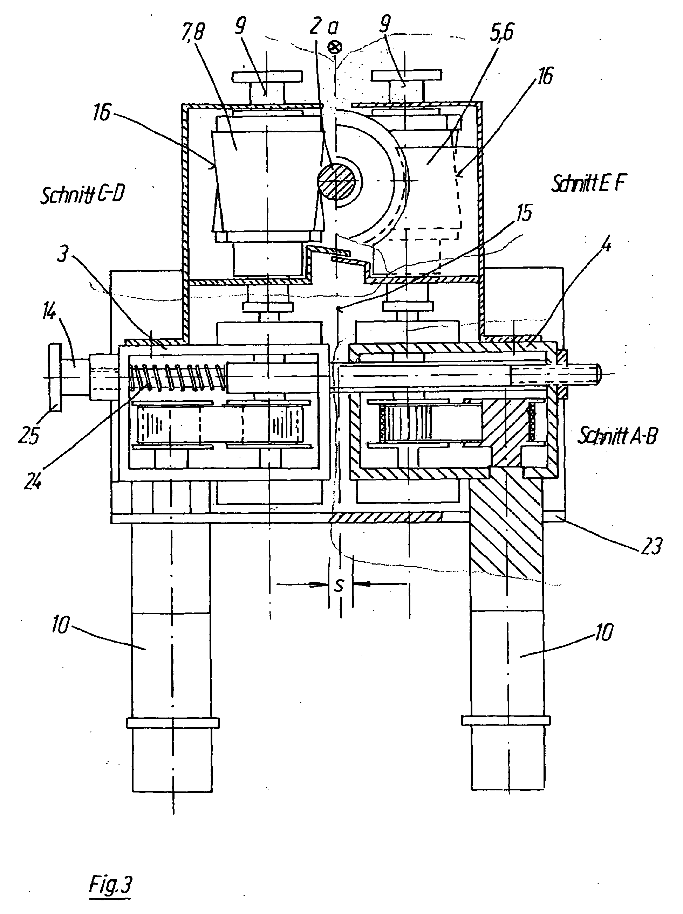

Dabei sind je zwei das Endoskop 2 klemmende Walzen - einmal die Walzen 5 und 7 und einmal die Walzen 6 und 8 - hinsichtlich ihres Umfangskonus gegensinnig angeordnet, wie es aus Fig. 3 hervorgeht. Andererseits sind auch die benachbart angeordneten Walzen 5 und 6 sowie 7 und 8 hinsichtlich ihres Umfangskonus gegensinnig angeordnet. Das Endoskop 2 wird hierdurch zentriert in der skizzierten Lage gehalten.

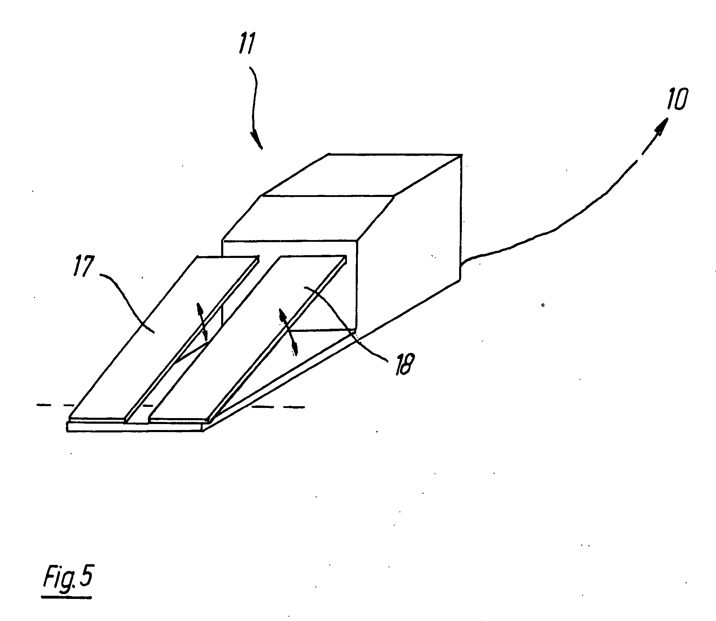

Die Walzen 5, 6, 7, 8 stehen über Elektroantriebe 10 in Getriebe-Verbindung, d. h. bei der Betätigung der Elektroantriebe 10 drehen sich die Walzen 5, 6, 7, 8. Die Elektroantriebe 10 werden dabei von einer nicht dargestellten Steuerung angesteuert, die wiederum ihre Steuersignale von einem Pedal 11, s. Fig. 5, erhält, das vom Arzt betätigt wird.

Das Pedal 11 weist im Ausführungsbeispiel zwei Fußtritte 17 und 18 auf, nämlich eines für den Vortrieb des Endoskops 2 und eines für dessen Rückzug. Die Fußtritte können dabei an die Steuerung einen vom Betätigungswinkel des Fußtritts 17, 18 (s. eingetragene Pfeile in Fig. 5) proportionalen Wert liefern, so dass die Elektroantriebe 10 entsprechend dem Betätigungswinkel der Fußtritt 17, 18 mehr oder weniger stark angetrieben werden.

Das zwei Fußtritte 17, 18 aufweisende Pedal 11 ist damit also feinsteuerbar wie ein "Gaspedal", der rechte Fußtritt 18 kann beispielsweise zum Vortreiben des Endoskops 2 dienen, der linke Fußtritt 17 entsprechend zum Zurückziehen.

Es kann auch vorgesehen sein, dass das Pedal 11 nur einen einzigen Fußtritt hat, der beispielsweise durch eine Schwenkbewegung um die Vertikalachse von Vortrieb auf Rückzug des Endoskops schaltet.

Ein sehr vorteilhaftes Merkmal des vorgeschlagenen Antriebs für das Endoskop ist die einstellbare bzw. vorgebbare Begrenzung der axialen Vorschubskraft F, die die Antriebswalzen 5, 6, 7, 8 auf das Endoskop 2 ausüben. Namentlich kann eine maximale Kraft Fₘₐₓ der Steuerung vorgegeben werden, die nicht überschritten werden darf. Hierzu weist der Antrieb 1 Kraftmessmittel 12 auf, die eine an sich bekannte Brückenschaltung von Dehnmessstreifen (DMS) aufweisen. Wie beispielsweise in Fig. 1 zu sehen ist, kann damit die Kraft F gemessen werden, mit der das Endoskop 2 in axiale Richtung a vorgetrieben wird. In dieser Figur ist auch schematisch dargestellt, dass Anzeigemittel 13 vorhanden sind, mit der die aktuelle Vorschubkraft F darstellbar ist.

Eine elektronische Messung der Vorschubkraft ist grundsätzlich auch über die Messung einer Widerstandsänderungen der Elektroantriebe 10 möglich.

Weiterhin ist die Kraftmessung grundsätzlich auch dadurch möglich, dass anstatt der Druckmessung am Gehäuse Drucksensoren im Bereich der Endoskopspitze angeordnet sind, die ein Signal an die Anzeigemittel 13 übermitteln.

Bei den Anzeigemitteln 13 ist insbesondere an eine Anzahl Leuchtdioden 26 gedacht, von denen umso mehr eingeschaltet werden, je größer die Kraft wird. Die ersten Leuchtdioden sind dabei grün, die mittleren gelb und die letzten rot, so dass sofort aufgrund der Farbe der Leuchtdioden ersichtlich ist, wie hoch qualitativ die Vorschubkraft F ist. Beispielsweise kann vorgesehen werden, dass Assistenzärzte nur im "grünen" Bereich arbeiten dürfen, dass Oberärzte bis hin zum "gelben" Bereich den Antrieb benutzen dürfen und dass nur Chefärzte in den roten Bereich vorstoßen dürfen, da dann von besonderen Verhältnissen in der Körperhöhle auszugehen ist.

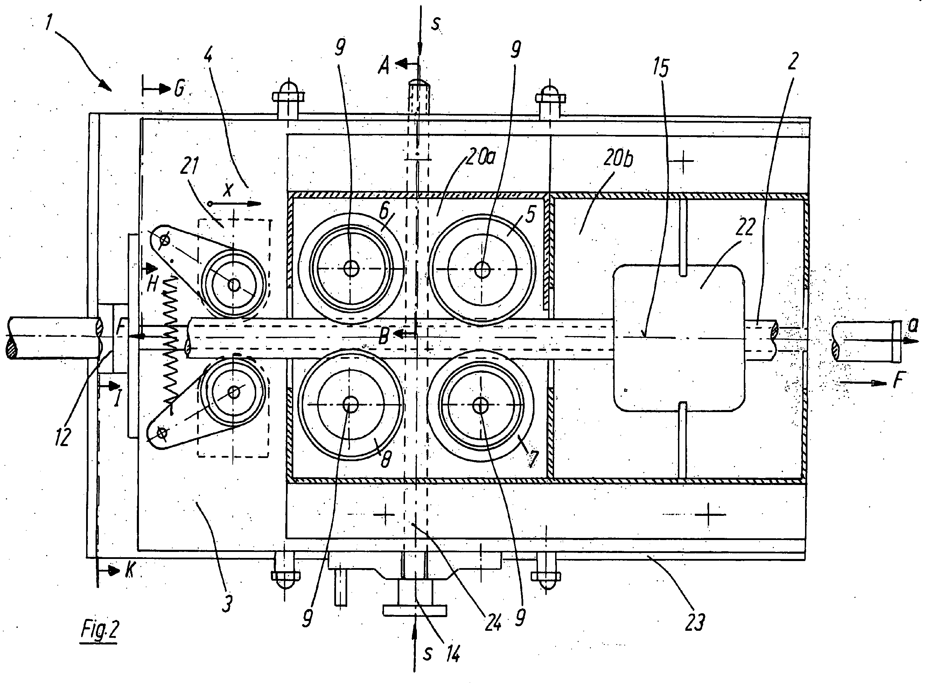

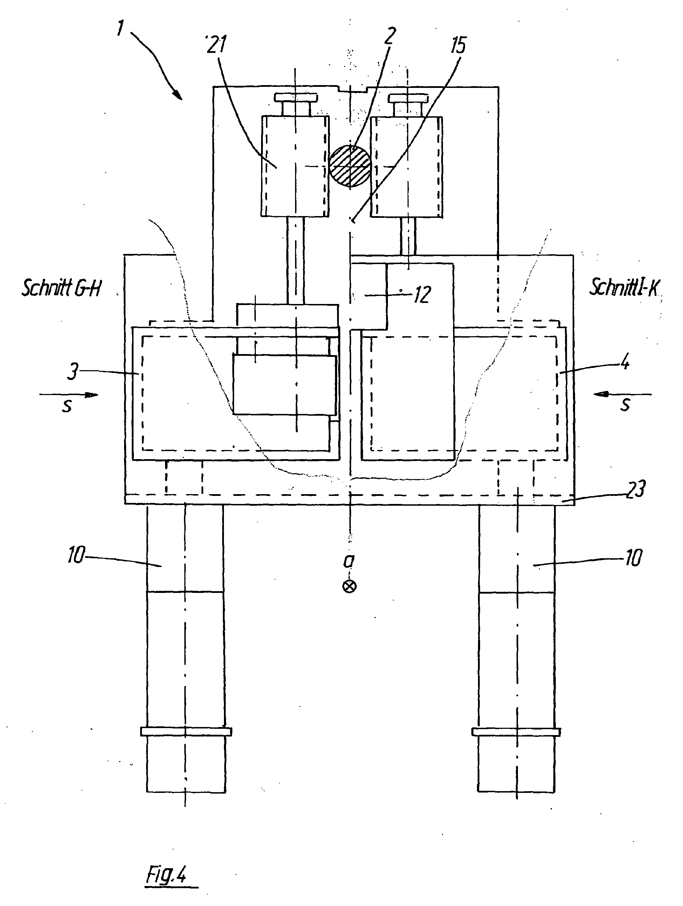

Den Einführweg x des Endoskops 2 (Vorschub- bzw. Rückzugsweg) in die Körperhöhle kann durch Wegmessmittel 21, s. insbesondere Figuren 2 und 4, ermittelt werden, wobei diese aus mindestens einer federvorgespannten Rolle bestehen können, die am Außenumfang des Endoskops 2 anliegt.

Die Sauberhaltung des Antriebs 1 wird durch ein Hygienemodul 19 vereinfacht (schematisch angedeutet in Fig. 2), womit es möglich wird, alle vier Antriebswalzen 5, 6, 7, 8 als Ganzes abzunehmen und zu sterilisieren. Hierzu können Schnellkupplungsmittel (nicht näher dargestellt) zwischen den Antriebswalzen und den Elektroantrieben 10 vorgesehen werden.

Unter den Antriebswalzen 5, 6, 7, 8 ist eine (zweiteilig ausgebildete) Auffangschale 20 (Teile 20a und 20b in Fig. 2) angeordnet, die die hygienische Handhabung des Antriebs erleichtert.

Die der Hygiene dienende Auffangschale 20 hat elastische Durchführungsabdichtungen, die den Weg in Verstellrichtung s quer zur axialen Richtung a des Endoskops 2 beim Einspannen desselben und den Weg in axiale Richtung a zur Kraftmessung mitmachen müssen.

Ebenfalls der Hygiene dienen Waschmittel 22, die in den Figuren nur schematisch dargestellt sind. Hierbei kann es sich um Sprühdüsen handeln, die den Endoskopumfang mit einer Reinigungsflüssigkeit besprühen. Die Waschmittel können auch Abstreifringe zur Reinigung des zurückgeführten und möglicherweise von Stuhl bzw. Schleimresten verunreinigten Endoskops 2 aufweisen.

Eine wichtige Funktionalität ist dadurch gegeben, dass das Endoskop 2 von oben zugänglich ist, also kein das Endoskop 2 abschließendes Gehäuse aufweist. Hiermit wird der schnelle und direkte Zugriff auf das Endoskop möglich.

Über ein Schnellentrastmittel (Schnellverschluss) des Schrauben-Gewinde-Elements 14 (nicht näher dargestellt) wird es möglich, mit einem Griff die Klemmung des Endoskops 2 aus der Spannung durch die Antriebswalzen 5, 6, 7, 8 zu lösen, so dass das Endoskop 2 bei Bedarf - in gewohnter händischer Weise - manipuliert werden kann.

Ein Ausführungsbeispiel sieht vor, dass der Gewindeeingriff zwischen Schraube und Mutter durch radiales Wegbewegen der Umfangssegmente eines in Umfangsrichtung geteilten Mutterelements aufgehoben wird, was durch Umlegen eines Hebelelements bewerkstelligt werden kann. Eine andere Lösung sieht vor, dass das Schrauben-Gewinde-Element 14 an einem axialen Ende an einem Verstellelement befestigt ist, das durch Umlegen eines Hebelelement die Schraube in Achsrichtung in Entspannrichtung verschiebt, so dass die Klemmung des Endoskops 2 aus der Spannung durch die Antriebswalzen 5, 6, 7, 8 aufgehoben wird.

### Bezueszeichenliste:

- 1: Antrieb
- 2: Endoskop
- 3: Trägerelement
- 4: Trägerelement
- 5: Antriebswalze
- 6: Antriebswalze
- 7: Antriebswalze
- 8: Antriebswalze
- 9: Achse
- 10: Elektroantrieb
- 11: Pedal
- 12: Kraftmessmittel
- 13: Anzeigemittel
- 14: Schrauben-Gewinde-Element
- 15: Symmetrieebene
- 16: Außenumfang
- 17: Fußtritt
- 18: Fußtritt
- 19: Hygienemodul
- 20: Auffangschale
- 21: Wegmessmittel
- 22: Waschmittel
- 23: Grundgerüst
- 24: Gewindespindel mit Rückstellfeder

- 25: Handrad
- 26: Leuchtdiode

- a: axiale Richtung
- s: Verstellrichtung
- F: axiale Kraft
- Fₘₐₓ: maximale axiale Kraft
- x: Vorschub- und Rückzugsweg

## Patentansprüche

1. Antrieb (1) für ein zumindest abschnittsweise rohrförmig ausgebildetes, flexibles Endoskop (2) zu dessen Bewegung in axialer Richtung (a),
wobei der Antrieb (1) mindestens zwei Trägerelemente (3, 4) aufweist, die relativ zueinander senkrecht zur axialen Richtung (a) des Endoskops (2) in einer Verstellrichtung (s) bewegbar und in einer gewünschten Position feststellbar sind,
wobei auf jedem der mindestens zwei Trägerelemente (3, 4) je mindestens eine Antriebswalze (5, 6, 7, 8) angeordnet ist, deren Achse (9) senkrecht zur axialen Richtung (a) des Endoskops (2) und senkrecht zur Verstellrichtung (s) angeordnet ist, wobei die mindestens eine Antriebswalze (5, 6, 7, 8) zum reibschlüssigen Antrieb des zwischen den Antriebswalzen (5, 6, 7, 8) angeordneten Endoskops (2) ausgebildet sind, und
wobei zumindest eine der Antriebswalzen (5, 6, 7, 8), vorzugsweise alle Antriebswalzen, von einem steuer- oder regelbaren Elektroantrieb (10) angetrieben wird, wobei der Elektroantrieb (10) mit einem Pedal (11) in Verbindung steht, über dessen Betätigungsweg und/oder Betätigungsrichtung die Drehgeschwindigkeit und/oder Drehrichtung der Antriebswalzen (5, 6, 7, 8) beeinflusst wird,
**dadurch gekennzeichnet,**
**dass** die mindestens zwei Trägerelemente (3, 4) relativ zueinander mittels eines Schrauben-Gewinde-Elements (14) bewegbar und/oder einstellbar sind, wobei das Schrauben-Gewinde-Element (14) eine Entraststellung oder Entrastmittel aufweist, in der die oder mit der die mindestens zwei Trägerelemente (3, 4) ohne Betätigung des Schrauben-Gewinde-Elements (14) voneinander entfernbar sind.

2. Antrieb nach Anspruch 1, **dadurch gekennzeichnet, dass** er zur Bewegung des Endoskops (2) in beide Achsrichtungen ausgebildet ist.

3. Antrieb nach Anspruch 1 oder 2, **dadurch gekennzeichnet, dass** das Pedal (11) und/oder der steuer- oder regelbaren Elektroantrieb (10) zur stufenlosen Bewegung des Endoskops (2) ausgebildet sind.

4. Antrieb nach einem der Ansprüche 1 bis 3, **gekennzeichnet durch** Kraftmessmittel (12), mit denen die von der mindestens einen Antriebswalze (5, 6, 7, 8) auf das Endoskop (2) aufgebrachte axiale Kraft (F) messbar ist.

5. Antrieb nach Anspruch 4, **dadurch gekennzeichnet, dass** die Kraftmessmittel (12) mindestens einen Dehnmessstreifen (DMS) aufweisen.

6. Antrieb nach Anspruch 4 oder 5, **gekennzeichnet durch** Anzeigemittel (13) zum Anzeigen der von der mindestens einen Antriebswalze (5, 6, 7, 8) auf das Endoskop (2) aufgebrachten axialen Kraft.

7. Antrieb nach Anspruch 6, **dadurch gekennzeichnet, dass** die Anzeigemittel (13) optische Elemente aufweisen, insbesondere Leuchtdioden, die bereichsweise unterschiedliche Farben haben.

8. Antrieb nach Anspruch 6, **dadurch gekennzeichnet, dass** die Anzeigemittel (13) akustische Elemente aufweisen.

9. Antrieb nach Anspruch 6, **dadurch gekennzeichnet, dass** die Anzeigemittel (13) Vibrationen aussendende Elemente aufweisen.

10. Antrieb nach einem der Ansprüche 4 bis 9, **dadurch gekennzeichnet, dass** die Kraftmessmittel (12) mit einem Schaltelement in Verbindung stehen, das zur Abschaltung des Elektroantriebs (10) bei Überschreitung eines vorgegebenen Wertes (Fₘₐₓ) der von der mindestens einen Antriebswalze (5, 6, 7, 8) auf das Endoskop (2) aufgebrachten axialen Kraft (F) ausgebildet ist.

11. Antrieb nach einem der Ansprüche 1 bis 10, **dadurch gekennzeichnet, dass** das Schrauben-Gewinde-Element (14) zur spiegelbildlichen Betätigung der mindestens zwei Trägerelemente (3, 4) zu einer Symmetrieebene (15) ausgebildet ist.

12. Antrieb nach einem der Ansprüche 1 bis 11, **dadurch gekennzeichnet, dass** die Antriebswalzen (5, 6, 7, 8) eine Ummantelung mit einem Material mit hohem Reibungskoeffizienten aufweisen.

13. Antrieb nach Anspruch 12, **dadurch gekennzeichnet, dass** das Material Gummi ist.

14. Antrieb nach einem der Ansprüche 1 bis 13, **dadurch gekennzeichnet, dass** die Antriebswalzen (5, 6, 7, 8) einen konischen Außenumfang (16) aufweisen.

15. Antrieb nach Anspruch 14, **dadurch gekennzeichnet, dass** je zwei zusammenwirkende Antriebswalzen (5, 7; 6, 8) so angeordnet sind, dass ihre konischen Außenumfänge (16) gegensinnig orientiert sind.

16. Antrieb nach einem der Ansprüche 1 bis 15, **dadurch gekennzeichnet, dass** das Pedal (11) zwei Fußtritte (17, 18) aufweist, wobei einer für den Vortrieb des Endoskops (2) in die eine axiale Richtung und einer für den Rückzug des Endoskops (2) in die andere axiale Richtung vorgesehen ist.

17. Antrieb nach einem der Ansprüche 1 bis 16, **dadurch gekennzeichnet, dass** die Trägerelemente (3, 4) samt Antriebswalzen (5, 6, 7, 8) frei zugänglich sind.

18. Antrieb nach einem der Ansprüche 1 bis 17, **dadurch gekennzeichnet, dass** zumindest die Antriebswalzen (5, 6, 7, 8) mit der restlichen Antriebsvorrichtung koppelbar und von dieser entkoppelbar ausgebildet sind.

19. Antrieb nach Anspruch 18, **dadurch gekennzeichnet, dass** die Antriebswalzen (5, 6, 7, 8) auf einem Hygienemodul (19) angeordnet sind.

20. Antrieb nach Anspruch 19, **dadurch gekennzeichnet, dass** unter dem Hygienemodul (19) eine Auffangschale (20) angeordnet ist, die zu den Trägerelementen (3, 4) abgedichtet ist.

21. Antrieb nach einem der Ansprüche 1 bis 20, **dadurch gekennzeichnet, dass** sie Wegmessmittel (21) zum Messen des Vorschub- und Rückzugswegs (x) des Endoskops (2) aufweist.

22. Antrieb nach einem der Ansprüche 1 bis 21, **gekennzeichnet durch** Waschmittel (22) zum Reinigen des Endoskops (2).

## Claims

1. Drive (1) for a flexible endoscope (2), which is of tubular configuration in at least some sections, for the purpose of moving said endoscope (2) in its axial direction (a),
wherein the drive (1) comprising at least two support elements (3, 4) which can be moved relative to one another in a displacement direction (s) perpendicular to the axial direction (a) of the endoscope (2) and can be fixed in a desired position,
wherein on each of the at least two support elements (3, 4) there is arranged at least one drive roller (5, 6, 7, 8) whose axis (9) is arranged perpendicular to the axial direction (a) of the endoscope (2) and perpendicular to the displacement direction (s), wherein the at least one drive roller (5, 6, 7, 8) being designed to frictionally engage and drive the endoscope (2) arranged between the drive rollers (5, 6, 7, 8), and
wherein at least one of the drive rollers (5, 6, 7, 8), preferably all the drive rollers, being driven by a controllable or feed-back-controllable electric drive (10), wherein said electric drive (10) being connected to a pedal (11) whose actuation travel and/or actuation direction influences the speed of rotation and/or direction of rotation of the drive rollers (5, 6, 7, 8),
**characterized in**
**that** the at least two support elements (3, 4) can be moved and/or adjusted relative to one another by means of a screw-thread element (14), wherein the screw-thread element (14) having a release position or release means in which or by which the at least two support elements (3, 4) can be distanced from one another without actuation of the screw-thread element (14).

2. Drive according to claim 1, **characterized in that** it is designed for moving the endoscope (2) in both axial directions.

3. Drive according to claim 1 or 2, **characterized in that** the pedal (11) and/or the controllablc or feed-back-controllable electric drive (10) are designed for stepless movement of the endoscope (2).

4. Drive according to one of claims 1 to 3, **characterized by** force sensors (12) with which it is possible to measure the axial force (F) applied to the endoscope (2) by the at least one drive roller (5, 6, 7, 8).

5. Drive according to claim 4, **characterized in that** the force sensors (12) comprise at least one strain gauge.

6. Drive according to claim 4 or 5, **characterized by** display means (13) for displaying the axial force applied to the endoscope (2) by the at least one drive roller (5, 6, 7, 8).

7. Drive according to claim 6, **characterized in that** the display means (13) have optical elements, in particular light-emitting diodes, which have different colours in different ranges.

8. Drive according to claim 6, **characterized in that** the display means (13) comprise acoustic elements.

9. Drive according to claim 6, **characterized in that** the display means (13) comprise elements emitting vibrations.

10. Drive according to one of claims 4 to 9, **characterized in that** the force sensors (12) are connected to a switch element which is designed to switch off the electric drive (10) if a predetermined value (Fₘₐₓ) of the axial force (F) applied to the endoscope (2) by the at least one drive roller (5, 6, 7, 8) is exceeded.

11. Drive according to one of claims 1 to 10, **characterized in that** the screw-thread element (14) is designed for mirror-image actuation of the at least two support elements (3, 4) with respect to a plane of symmetry (15).

12. Drive according to one of claims 1 to 11, **characterized in that** the drive rollers (5, 6, 7, 8) comprise a covering made from a material with a high coefficient of friction.

13. Drive according to claim 12, **characterized in that** the material is rubber.

14. Drive according to one of claims 1 to 13, **characterized in that** the drive rollers (5, 6, 7, 8) have a conical outer periphery (16).

15. Drive according to claim 14, **characterized in that** every two cooperating drive rollers (5, 7; 6, 8) are arranged in such a way that their conical outer peripheries (16) are oriented in opposite directions.

16. Drive according to one of claims 1 to 15, **characterized in that** the pedal (11) has two foot panels (17, 18), wherein one being provided for advancing the endoscope (2) in one axial direction, and one being provided for withdrawing the endoscope (2) in the other axial direction.

17. Drive according to one of claims 1 to 16, **characterized in that** the support elements (3, 4) including drive rollers (5, 6, 7, 8) are freely accessible.

18. Drive according to one of claims 1 to 17, **characterized in that** at least the drive rollers (5, 6, 7, 8) are designed such that they can be coupled to the rest of the drive device and can be uncoupled from it.

19. Drive according to claim 18, **characterized in that** the drive rollers (5, 6, 7, 8) are arranged on a hygiene module (19).

20. Drive according to claim 19, **characterized in that** a collecting dish (20) is arranged under the hygiene module (19) and is sealed off from the support elements (3, 4).

21. Drive according to one of claims 1 to 20, **characterized in that** it comprises position sensors (21) for measuring the advance and withdrawal (x) of the endoscope (2).

22. Drive according to one of claims 1 to 21, **characterized by** wash means (22) for cleaning the endoscope (2).

## Revendications

1. Entraînement (1) pour un endoscope (2) flexible réalisé au moins en partie sous forme tubulaire, en vue de son déplacement dans la direction axiale (a),
l'entraînement (1) présentant au moins deux éléments de support (3, 4) qui peuvent être déplacés l'un par rapport à l'autre perpendiculairement à la direction axiale (a) de l'endoscope (2) dans une direction de réglage (s) et qui peuvent être fixés dans une position souhaitée,
au moins un rouleau d'entraînement (5, 6, 7, 8) étant disposé sur chacun des au moins deux éléments de support (3, 4), dont l'axe (9) est disposé perpendiculairement à la direction axiale (a) de l'endoscope (2) et perpendiculairement à la direction de réglage (s), l'au moins un rouleau d'entraînement (5, 6, 7, 8) étant réalisé pour l'entraînement par engagement de friction de l'endoscope (2) disposé entre les rouleaux d'entraînement (5, 6, 7, 8), et
au moins l'un des rouleaux d'entraînement (5, 6, 7, 8), et de préférence tous les rouleaux d'entraînement, étant entraînés par un entraînement électrique commandable ou réglable (10), l'entraînement électrique (10) étant en liaison avec une pédale (11) dont la course d'actionnement et/ou la direction d'actionnement influence la vitesse de rotation et/ou le sens de rotation des rouleaux d'entraînement (5, 6, 7, 8),
**caractérisé en ce que**
les au moins deux éléments de support (3, 4) peuvent être déplacés et/ou ajustés l'un par rapport à l'autre au moyen d'un élément de vis fileté (14), l'élément de vis fileté (14) présentant une position de désencliquetage ou un moyen de désencliquetage dans laquelle ou avec lequel les au moins deux éléments de support (3, 4) peuvent être séparés l'un de l'autre sans actionnement de l'élément de vis fileté (14) .

2. Entraînement selon la revendication 1, **caractérisé en ce qu'**il est réalisé en vue du déplacement de l'endoscope (2) dans les deux directions axiales.

3. Entraînement selon la revendication 1 ou 2, **caractérisé en ce que** la pédale (11) et/ou l'entraînement électrique commandable ou réglable (10) sont réalisés en vue du déplacement continu de l'endoscope (2).

4. Entraînement selon l'une quelconque des revendications 1 à 3, **caractérisé par** des moyens de mesure de force (12) avec lesquels la force (F) axiale appliquée par l'au moins un rouleau d'entraînement (5, 6, 7, 8) sur l'endoscope (2) peut être mesurée.

5. Entraînement selon la revendication 4, **caractérisé en ce que** les moyens de mesure de force (12) présentent au moins un ruban de mesure d'allongement (DMS).

6. Entraînement selon la revendication 4 ou 5, **caractérisé par** des moyens d'indication (13) pour indiquer la force axiale appliquée par l'au moins un rouleau d'entraînement (5, 6, 7, 8) sur l'endoscope (2).

7. Entraînement selon la revendication 6, **caractérisé en ce que** les moyens d'affichage (13) présentent des éléments optiques, en particulier des diodes électroluminescentes, dont les parties ont différentes couleurs.

8. Entraînement selon la revendication 6, **caractérisé en ce que** les moyens d'affichage (13) présentent des éléments acoustiques.

9. Entraînement selon la revendication 6, **caractérisé en ce que** les moyens d'entraînement (13) présentent des éléments produisant des vibrations.

10. Entraînement selon l'une quelconque des revendications 4 à 9, **caractérisé en ce que** les moyens de mesure de force (12) sont en liaison avec un élément de commutation qui est réalisé en vue de couper l'entraînement électrique (10) en cas de dépassement d'une valeur prédéterminée (Fₘₐₓ) de la force (F) axiale appliquée par l'au moins un rouleau d'entraînement (5, 6, 7, 8) sur l'endoscope (2).

11. Entraînement selon l'une quelconque des revendications 1 à 10, **caractérisé en ce que** l'élément de vis fileté (14) est réalisé en vue de l'actionnement symétrique des au moins deux éléments de support (3, 4) par rapport à un plan de symétrie (15).

12. Entraînement selon l'une quelconque des revendications 1 à 11, **caractérisé en ce que** les rouleaux d'entraînement (5, 6, 7, 8) présentent une enveloppe avec un matériau ayant un coefficient de friction élevé.

13. Entraînement selon la revendication 12, **caractérisé en ce que** le matériau est du caoutchouc.

14. Entraînement selon l'une quelconque des revendications 1 à 13, **caractérisé en ce que** les rouleaux d'entraînement (5, 6, 7, 8) présentent une périphérie extérieure conique (16).

15. Entraînement selon la revendication 14, **caractérisé en ce que** deux rouleaux d'entraînement coopérants (5, 7 ; 6, 8) sont à chaque fois disposés de telle sorte que leurs périphéries extérieures coniques (16) soient orientées en sens inverse.

16. Entraînement selon l'une quelconque des revendications 1 à 15, **caractérisé en ce que** la pédale (11) présente deux appuis de pédale (17, 18), l'un étant prévu pour l'avance de l'endoscope (2) dans une direction axiale et l'autre étant prévu pour le retour de l'endoscope (2) dans l'autre direction axiale.

17. Entraînement selon l'une quelconque des revendications 1 à 16, **caractérisé en ce que** les éléments de support (3, 4) ainsi que les rouleaux d'entraînement (5, 6, 7, 8) sont librement accessibles.

18. Entraînement selon l'une quelconque des revendications 1 à 17, **caractérisé en ce qu'**au moins les rouleaux d'entraînement (5, 6, 7, 8) sont réalisés de manière à pouvoir être accouplés avec le reste du dispositif d'entraînement et de manière à pouvoir être désaccouplés de celui-ci.

19. Entraînement selon la revendication 18, **caractérisé en ce que** les rouleaux d'entraînement (5, 6, 7, 8) sont disposés sur un module hygiénique (19).

20. Entraînement selon la revendication 19, **caractérisé en ce qu'**une coque de réception (20) est disposée sous le module hygiénique (19), laquelle est étanchéifiée par rapport aux éléments de support (3, 4).

21. Entraînement selon l'une quelconque des revendications 1 à 20, **caractérisé en ce qu'**il présente des moyens de mesure de distance (21) pour mesurer la course d'avance et de retour (x) de l'endoscope (2).

22. Entraînement selon l'une quelconque des revendications 1 à 21, **caractérisé par** des moyens de lavage (22) pour le nettoyage de l'endoscope (2) .
